## Europäisches Patentamt

## European Patent Office

## Office européen des brevets

(11) Publication number: **0 221 561**
**A2**

(12) # EUROPEAN PATENT APPLICATION

(21) Application number: **86115407.8**

(22) Date of filing: **06.11.86**

(51) Int. Cl.⁴: **C 07 K 15/06**
**C 07 K 15/14, C 12 N 5/00**
**C 07 K 15/00, C 12 P 21/00**
**A 61 K 39/395, A 61 K 49/02**
**G 01 N 33/48, G 01 N 33/574**
**G 01 N 33/543, G 01 N 33/5-77**
**//(C12P21/00, C12R1:91)**

(30) Priority: **07.11.85 US 795981**

(43) Date of publication of application:
**13.05.87 Bulletin 87/20**

(84) Designated Contracting States:
**AT BE CH DE ES FR GB GR IT LI LU NL SE**

(71) Applicant: **The Trustees of Columbia University in the City of New York**
**Broadway and West 116th Street**
**New York, NY 10027(US)**

(72) Inventor: **Mesa-Tejada, Ricardo**
**19 West 85th Street**
**New York New York 10024(US)**

(74) Representative: **Henkel, Feiler, Hänzel & Partner**
**Möhlstrasse 37**
**D-8000 München 80(DE)**

(54) Antigen indicative of human breast cancer and assays based thereon.

(57) This invention concerns a glycoprotein which is useful as an indicator of human breast cancer. This glycoprotein is derived from human breast carcinomas or metastases thereof and has a molecular weight of 225-250 kilodaltons. It is specifically recognized by the monoclonal antibody deposited under ATCC Accession No. HB 8936 (CU26).

This invention also concerns assays for diagnosing and monitoring human breast cancer utilizing monoclonal antibodies directed to this glycoprotein.

EP 0 221 561 A2

## ANTIGEN INDICATIVE OF HUMAN BREAST CANCER
## AND ASSAYS BASED THEREON

The invention described herein was made with government support under Grant CA 32984 from the National Cancer Institute, National Institute of Health, U.S. Department of Health and Human Services. The U.S. Government has certain rights in this invention.

### BACKGROUND OF THE INVENTION

Throughout this application, various publications are referenced to provide background information concerning the state of the art as known to those of ordinary skill therein as of the date of the invention disclosed and claimed herein. In the text of this application, these publications will be referred to by full citations. The disclosures of these references in their entireties are hereby incorporated by reference into the present application.

The longtime research interest in the etiologic role of the mouse mammary tumor virus (MMTV) in mouse mammary tumors and a hypothetical similar relationship in human breast cancer led to the discovery of an immunocytochemically detectable glycoprotein in human breast cancer tissues that was specifically immunologically related to the major envelope glycoprotein of the mouse mammary tumor virus (Mesa-Tejada, R. et al., Proc. Nat. Acad Sci. USA 75: 1529, (1978). Subsequent attempts to further characterize and isolate the human crossreactive antigen led to the selection of the T47D cell line, a human breast carcinoma cell line established

0221561

from cells isolated from the pleural effusion of a breast carcinoma patient, and specifically clone 11, as the most convenient potential source of this antigen. This cell line consistently produces particles that possess the physical and biochemical characteristics of RNA tumor viruses, and later studies established that the crossreactive antigen is present in the fraction corresponding to the region in a sucrose density gradient in which these particles are found (Keydar, I. et al., Proc. Nat. Acad. Sci. USA 81: 4188 (1984)). This antigen-enriched fraction is known as the viral region (VR).

Spiegelman (U.S. Patent No. 4,379,839) discloses a method for detecting breast cancer by immunologically assaying samples for breast cancer specific viral related proteins utilizing the cross-reactivity of the viral related protein with antibodies directed to Mason-Pfizer Monkey Virus or murine mammary tumor virus.

Mesa-Tejada et al., Proceedings of the Biennial International Breast Cancer Research Conference, Denver, Colorado, March 20-24, 1983 Abstract 97, describe studies of monoclonal antibodies to RNA virus-like particles from the T47D breast carcinoma cell line. They reported that the ascites form of two monoclonal antibodies ($ASC_218$, $ASC_246$), to virus-like particles isolated from the T47D clone 11 cell gave positive staining of malignant breast carcinoma tissue.

Ohno et al., Proceedings of the Biennial International Breast Cancer Research Conference, Denver, Colorado, March 20-24, 1983, Abstract 96 describe two monoclonal antibodies $S_1C_1$ 18 and $S_1C_1$ 46 which recognize three proteins weighing approximately 280, 245 and 230 kilodaltons. The authors reported that the distribution of the stain produced by these antibodies in breast carcinomas suggested that an antigen was more likely to be found in the circulation of patients with invasive breast cancer and suggested that detection of this antigen in the serum of such patients might have diagnostic significance.

Recently, Nepo et al., the Breast Cancer Research Conference, London, England, March 24-28, 1985 Abstract No. 6-13 and Cancer Drug Delivery, Vol. 2, p. 228, September 12-13, 1985 reported studies involving the uptake and intracellular distribution of a daunorubicin (DNR)-CU18 antibody conjugate by clone 11 T47D cells.

Jonassen and Mesa-Tejada, Proceedings of the Biennial International Breast Cancer Research Conference, London, England, March 24-28, 1985 Abstract No. 4-33 reported on studies involving the immunocytochemical identification of cutaneous breast cancer metastases with monoclonal antibody CU18.

This invention involves the unexpected discovery that a specific glycoprotein, designated BCA-225, is shed or released from the surface of breast carcinomas into the circulating blood of persons having breast cancer. Utilizing this discovery, this invention provides simple non-invasive,. reliable and reproducible assays for diagnosing and monitoring breast cancer. This invention also provides chemically modified antibodies useful in immunotherapy and diagnostic imaging.

## SUMMARY OF THE INVENTION

A glycoprotein useful as an indicator of human breast cancer or mestatases thereof has been discovered and isolated. This glycoprotein designated BCA-225 is derived from human breast carcinomas and is a molecular species of about 225-250 kilodaltons. It is specifically recognized by the monoclonal antibody produced by the hybridoma cell line having ATCC Accession No. HB 8936 (CU26). Markers may be bound to this glycoprotein to facilitate its use in immunoassays.

This invention also encompasses a monoclonal antibody which specifically binds to this glycoprotein and the hybridoma which produces this monoclonal antibody. This hybridoma cell line has been deposited with the American Type Culture Collection under ATCC Accession No. HB 8936 (CU26) Markers may also be bound to this and other monoclonal antibodies which specifically bind to BCA-225.

The invention is further directed to assays for diagnosing human breast cancer. In these assays, the amount of the glycoprotein in a body fluid sample of defined volume from a subject, i.e., the concentration of glycoprotein in the sample, is determined. By comparing the amount determined with amounts or concentrations which indicate the presence or absence of human breast cancer, diagnosis may be effected.

In a presently preferred embodiment, the concentration of the glycoprotein in a serum sample from a patient is determined utilizing a monoclonal-monoclonal antibody sandwich assay in which one of the antibodies is immobilized on a solid support and the second antibody has bound to it a suitable marker.

This invention also provides monoclonal antibodies which specifically bind to the glycoprotein and to which chemotherapeutic agents, toxins or imageable entities are bound. Such antibodies may be used to deliver chemotherapeutic agents to, and to image, breast cancer cells.

BRIEF DESCRIPTION OF THE FIGURES

FIGURE 1

Reference Standard Curve for Detection of BCA-225 in Human Serum with CU18-CU46 Sandwich Assay

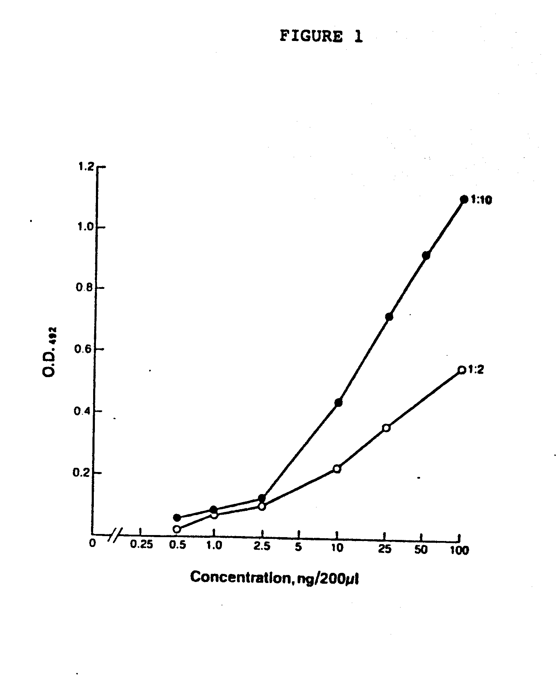

Figure 1 is a reference standard curve for detection of BCA-225 in human serum with the monoclonal CU18-CU46 sandwich ELISA. The results of the ELISA (optical density reading) of aliquots of pooled normal human serum (1:2, 1:10) containing increasing known concentrations of Sephacryl S-300 antigen with BCA-225 activity are shown. These results demonstrate the increased sensitivity of this assay at greater dilutions of serum and also demonstrate the sensitivity of the CU18-CU46 monoclonal antibody capture assay in detecting the antigen in amounts as low as 2 ng/ml.

FIGURE 2

Five-Layer CU18 Monoclonal-Polyclonal ELISA Comparison of BCA-225 Antigen Detection in PBS and in Different Dilutions of Serum

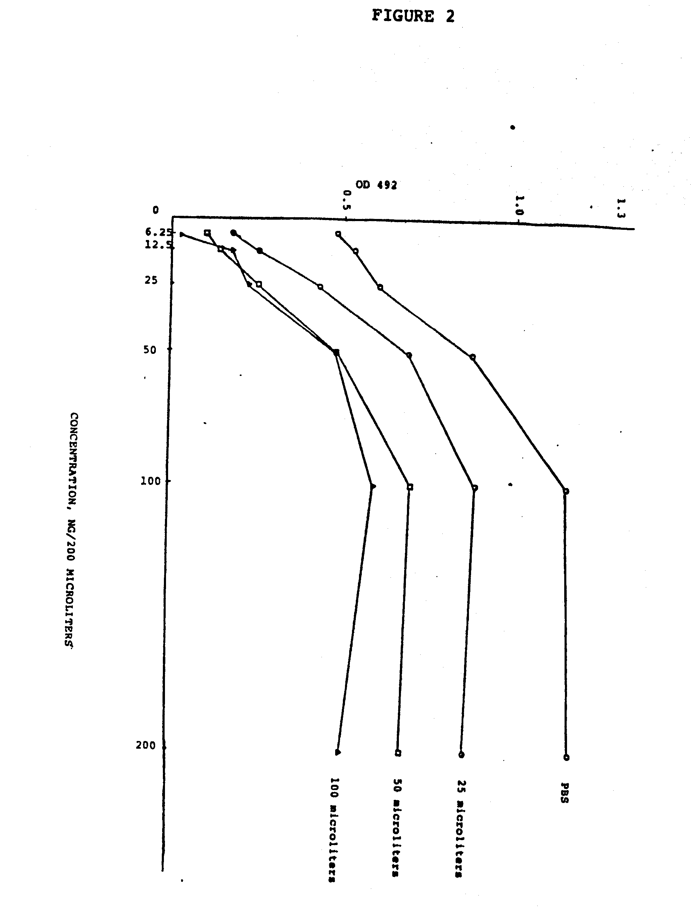

Figure 2 demonstrates the sensitivity of the five-layer monoclonal-polyclonal Enzyme Layer Immunosorbent Assay (ELISA) in detecting Sephacryl S-300 BCA-225 antigen in amounts as low as 6.25 ng/well. Since each well holds approximately 200 microliters, 6.25 ng/well is equivalent to 31.25 ng/ml. The monoclonal antibody used in this assay is CU18.

Figure 2 also shows that the sensitivity of the five-layer monoclonal-polyclonal immunoassay increases as smaller amounts of serum are used, as is at a maximum with Phosphate Buffered Saline (PBS).

PBS is indicated by the  O——O line;
25 ul of serum is indicated by the  O——O  line;
50 ul of serum is indicated by the  □——□  line; and
100 ul of serum is indicated by the  Δ——Δ  line.

A comparison of Figures 1 and 2 indicates that the monoclonal sandwich assay is more sensitive than the monoclonal-polyclonal assay and therefore better for detecting small amounts of antigen.

## FIGURE 3

### Clinical Data Showing the Correlation Between the Concentration of BCA-225 Antigen in the Sera of Patients and the Disease Status of the Patients

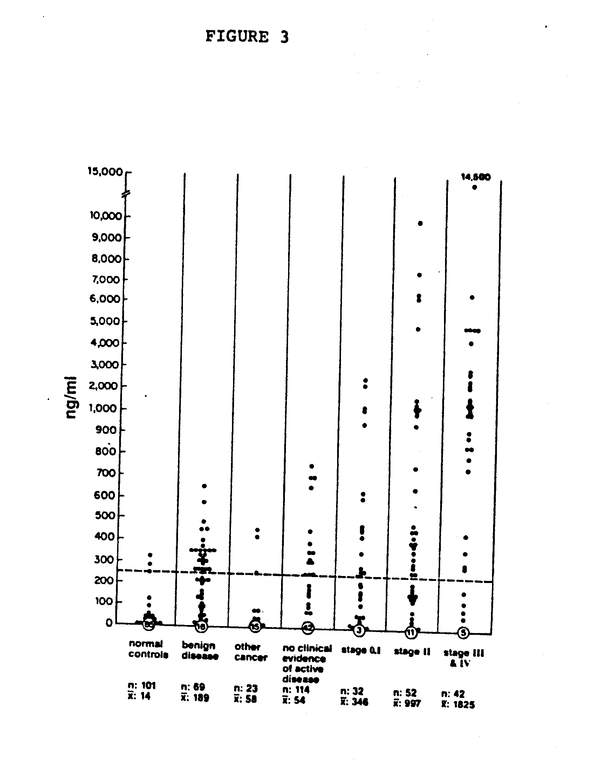

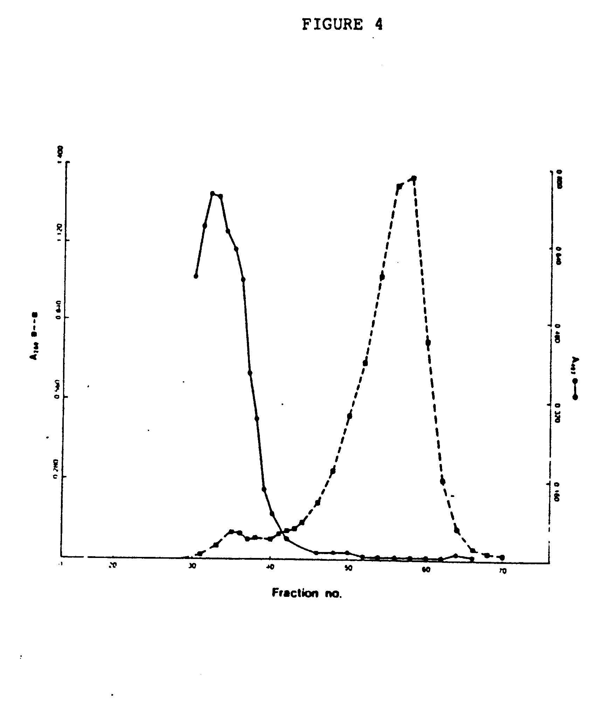

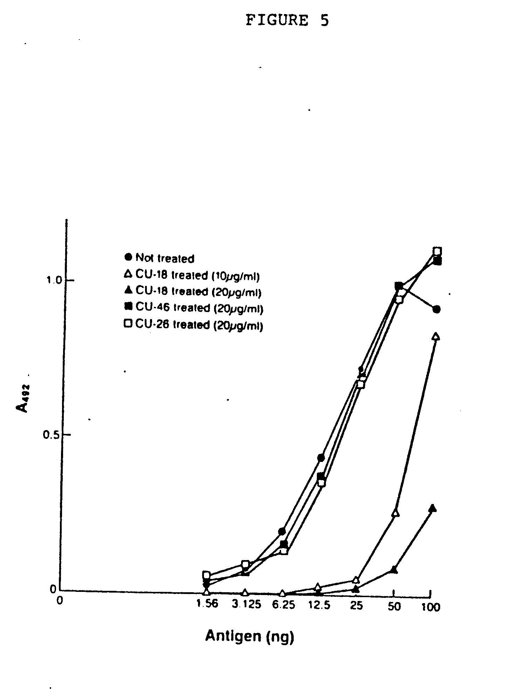

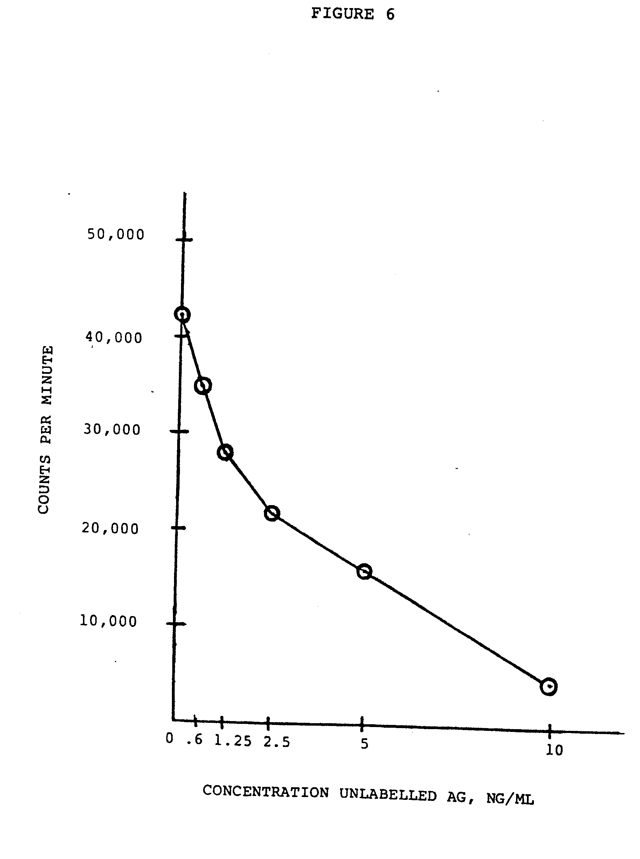

Figure 3 shows the correlation between the concentration of the Sephacryl S-300 BCA-225 antigen in ng/ml in the sera of patients and the state of human breast cancer disease in the patient. Figure 3 illustrates the concentration of the BCA-225 antigen values obtained in a series of 433 total patients sub-divided into seven clinically relevant groups as indicated. The circled numbers at the bottom of each column are the "0" values in that group, i.e., those patients in whose sera BCA-225 could not be detected. The dotted line at 250 ng/ml represents an arbitrary boundary between the "positive" (above) and "negative" (below) values. This value was arbitrarily chosen because it is approximately equal to the mean plus two standard deviations of the "Benign Breast Disease" group (second column).

FIGURE 4

Sephacryl S-300 Column Chromatography Separation of SUP-100 AS

Peak reactivity of BCA-225 (as determined by monoclonal ELISA) was found between fractions 30 and 40 (solid line) corresponding to only a small fraction of the total protein concentration (interrupted line) of the SUP-100 AS.

FIGURE 5

Blocking Effect of CU18

A direct ELISA demonstrates the blocking effect of unlabelled CU18 in the detection of BCA-225 by peroxidase-labelled CU18. Neither unlabelled CU26 nor CU46 demonstrate a blocking effect. This data suggests that the epitopes recognized by CU26 and CU46 on BCA-225 are different than that recognized by CU18.

FIGURE 6

Competitive Radioimmunoassay (RIA) for the Detection of BCA-225 in Human Serum

Tubes coated with CU18 were incubated with 150 ul of plasma (1:2) standards containing increasing known concentrations of unlabelled Sephacryl S-300 antigen, which had been incubated with 50 ul of $^{125}$I labelled Sephacryl S-300 antigen overnight at room temperature. The supernatant was then aspirated and the tubes were washed once with PBS-Tween and counted. The results

0221561

indicate dose-related competitive inhibition with total binding $(B_O/T)$ of approximately 25%.

DETAILED DESCRIPTION OF THE INVENTION

A glycoprotein, useful as an indicator of human breast cancer, has been discovered and isolated. This glycoprotein is found in, and derived from, the surface, cytoplasm and culture medium of a human breast carcinoma cell line, a primary human breast carcinoma and metastases thereof. It is a molecular species of about 225-250 kilodaltons as determined by gel electrophoresis and is designated BCA-225. The glycoprotein is an antigen to which the monoclonal antibody produced by the hybridoma cell line having ATCC Accession No. HB 8936 (CU26) specifically binds.

In order to facilitate the use of this glycoprotein in immunoassays, a marker may be bound to it by conventional methods known to those skilled in the art. The marker may be an enzyme which catalyzes a reaction which yields a detectable product, for example, horseradish peroxidase. The marker may also be a radioactive label, e.g., $^{125}I$, or a fluorescent label such as fluorescein isothiocyanate or rhodamine. Magnetic entities or paramagnetic elements such as iron (Fe), copper (Cu), manganese (Mn), gadolinium (Gd) or an imageable entity such as a radionuclide, for example $^{111}In$ may also be used as a marker.

The glycoprotein may be detected by monoclonal antibodies which bind specifically to it. Hybridomas which produce monoclonal antibodies having binding specificity to the glycoprotein have been deposited with the American Type Culture Collection in Rockville, Maryland 20852, U.S.A., under ATCC Accession Nos. HB 8936 (CU26), HB 8937 (CU46), HB 8940 CU18). These deposits were made pursuant to the provisions of the Budapest Treaty on the International Recognition of the Deposit of Microorganisms. These monoclonal antibodies which specifically bind to the glycoprotein-antigen BCA-225 may also be marked to facilitate their detection and therefore the detection of the glycoprotein in a sample taken from the subject. Markers which may be used with these antibodies include enzymes which catalyze a reaction which yields a detectable product, for example, horseradish peroxidase or radioactive or fluorescent labels, such as $^{125}$I or fluorescein isothiocyanate or rhodamine, respectively. Paramagnetic elements or imageable entities may also be used as antibody markers. Examples of such paramagnetic elements include iron (Fe), copper (Cu), manganese (Mn), and gadolinium (Gd). Examples of imageable entities include radionuclides such as $^{111}$In.

Methods for binding such markers to antibodies are conventional and well-known to persons skilled in the art. See, for instance, Krejcarek et al., BBRC 77: 581, which describes standard procedures for modifying the antibody molecule so it can chelate $^{111}$In and other metal ions.

The presence or absence of marked antibodies in the complex may be detected or measured by various techniques, depending on the particular immunoassay and marker used. For example, radioactive labels may be detected by scintillation counting, radiography, or other methods which can detect radioactive decay after separating the unreacted marked antibody. If an enzyme linked immunoassay is used, the presence of enzyme-labelled antibody in the antibody-antigen complex can be detected after removing unreacted enzyme-labelled antibody. A substrate is added which produces a colored precipitate on contact with the enzyme. The presence of color can be detected or measured spectrophotometrically, for example, to determine the presence or amount of antigen. Other detection methods known in the art may also be used including fluorimetry involving fluorogenic labels.

An assay for diagnosing human breast cancer utilizing the glycoprotein BCA-225 has been developed. In this assay, the amount of the glycoprotein BCA-225 present in a body fluid sample from the subject of defined volume is quantitatively determined and compared with predetermined amounts of BCA-225 in samples of the same volume which indicate the presence or absence of human breast cancer.

As used herein, the term "body fluids" includes whole blood, plasma, serum, cerebrospinal fluid, milk, colostrum and vaginal smears. Assays of serum samples are preferred.

Immunoassays for diagnosing human breast cancer in subjects have also been developed. In the immunoassays, the amount of the glycoprotein BCA-225 present in a body fluid sample of defined volume from the subject is quantitatively determined by contacting the sample with an antibody which specifically binds the glycoprotein. This amount of glycoprotein is then compared with predetermined amounts indicative of the presence or absence of human breast cancer in samples of the same volume and a diagnosis of human breast cancer made. Immunoassays wherein the antibody is marked are preferred.

In a preferred embodiment of the invention, human breast cancer may be diagnosed in a subject by an immunoassay which entails immobilizing a monoclonal antibody which specifically binds to an epitope on the glycoprotein on a support; then contacting the immobilized monoclonal antibody with a body fluid sample of defined volume from the subject under conditions permitting formation of a complex between the monoclonal antibody and the glycoprotein; removing the uncomplexed sample components; and quantitatively determining the amounts of glycoprotein present in the sample. This amount of glycoprotein is then compared with predetermined amounts of glycoprotein in samples of the same volume which predetermined amounts indicate the presence or absence of human cancer, thus enabling diagnosis of the presence or absence of the human breast cancer in the subject.

The support utilized in the immunoassay may be any suitable support for use in such assays including polystyrene, polystyrene bead and nitrocellulose supports.

Examples of preferred immunoassays which may be used for diagnosing human breast cancer include monoclonal-polyclonal immunoassays, monoclonal "sandwich" assays utilizing the same or different monoclonal antibodies directed to the glycoprotein, and monoclonal-monoclonal assays wherein the second monoclonal antibody specifically binds to the first antibody rather than the glycoprotein. Most preferably the amount of glycoprotein present in the sample is determined by a monoclonal "sandwich" immunoassay utilizing monoclonal antibodies which specifically bind to two different epitopes on the glycoprotein.

In a specific embodiment of the preferred immunoassay, the amount of glycoprotein present in the sample is determined by contacting the complex of first monoclonal antibody and glycoprotein with a detectable monoclonal antibody which specifically binds to a different epitope on the glycoprotein under conditions permitting the binding of the detectable monoclonal antibody to the complex; removing the unbound detectable monoclonal antibody; and quantitatively determining the amount of bound detectable monoclonal antibody and thereby the amount of glycoprotein in the complex.

The detectable antibody utilized in the preferred immunoassay may be an antibody linked to an enzyme which catalyzes a reaction which yields a detectable product, e.g., horseradish peroxidase or may be a radioactively or fluorescently labelled antibody, e.g., labelled with $^{125}I$ or fluorescein isothiocyanate or rhodamine. The detectable antibody may also be marked or labelled with a paramagnetic element such as iron, copper, manganese or gadolinium or may be linked to an imageable entity such as a radionuclide, e.g., $^{111}In$.

The invention also includes a method for diagnosing human breast cancer in the subject by contacting a body fluid sample of defined volume from the subject with an antibody to the glycoprotein of claim 1 under suitable conditions such that a detectable complex forms only if the glycoprotein is present in an amount indicative of human breast cancer, and detecting the complex. Utilizing this method of detecting breast cancer, no complex would form if the BCA-225 concentration in the sample was not high enough to indicate breast cancer.

A method for monitoring human breast cancer has also been developed. At various times, the amount of glycoprotein-BCA-225 present in body fluid samples of defined volumes from the subject is measured using the assays described above and the amounts compared. Increasing amounts of BCA-225 in the samples might indicate a recurrence of breast cancer.

A method for diagnosing or monitoring human breast cancer, wherein the antibody is linked to a magnetic entity to effect separation of malignant and benign cells is also part of this invention.

Methods of treating cancer by delivering chemotherapeutic agents or toxins to cancerous cells utilizing antibodies which specifically bind to BCA-225 are also provided by this invention. These methods involve contacting the cancerous cells with monoclonal antibodies to BCA-225 which are linked directly or indirectly to chemotherapeutic agents or toxins. An example of indirect linkage is where the chemotherapeutic agent or toxin is carried in a liposome bound to the antibody.

Standard procedures for linking chemotherapeutic agents and toxins to antibodies are well-known to those skilled in the art. See, for instance, Annals NY Acad. Sci. 446 (1985) wherein various methods to use liposomes attached to antibodies as drug carriers and radionuclide carriers are described.

Generally a chemotherapeutic agent may be any any chemical compound used for treatment. More specifically, the term refers to chemical compounds which are able to interfere with the genetics or metabolism of rapidly dividing cells. Examples of chemotherapeutic agents include daunorubicin.

Toxins are any poisons, especially proteins or conjugated protein substances produced by some higher plants, certain animals and pathogenic bacteria, that are highly toxic to other living organisms. Examples of toxins which may be employed in the immunotherapeutic aspects of this invention include ricin, abrin and gelonin.

Methods of imaging cancer cells especially breast cancer cells are also part of this invention. The cells are contacted with monoclonal antibodies to BCA-225 which are linked to an imageable entity and the imageable entity is then detected. Examples of suitable imageable entities include $^{99}$Technicium, $^{131}$I, radionuclides such as $^{111}$In or paramagnetic metals such as Fe, Cu, Mn or Gd. Methods of imaging include radiographic imaging and magnetic resonance imaging.

Competitive immunoassays which may also be used for diagnosing human breast cancer in subjects are also within the scope of this invention. A specific embodiment of a competitive immunoassay entails immobilizing on a support a monoclonal antibody which specifically binds to an epitope on the BCA-225 glycoprotein. The immobilized monoclonal antibody is then contacted with a body fluid sample of defined volume from a subject and a known amount of labelled BCA-225 glycoprotein, which amount is sufficient to bind all or most of the monoclonal antibody sites on the support, under conditions permitting the formation of a complex between the labelled glycoprotein, the monoclonal antibody and unlabelled glycoprotein. Uncomplexed components are then removed from the support. The amount of labelled glycoprotein in the complex is determined thereby enabling the amounts of unlabelled glycoprotein present in the samples to be determined. The amount of unlabelled glycoprotein is then compared with predetermined amounts indicative of the presence or absence of human breast cancer in samples of the same defined volume and the presence or absence or human breast cancer in the subject is diagnosed. Preferably, the labelled glycoprotein is radiolabelled, e.g., with $^{125}$I.

Applicant's invention also encompasses an immunoassay kit. The kit includes, _inter alia_, a support; a first monoclonal antibody directed to one epitope of the glycoprotein; a blocking solution to block sites on the support where no first monoclonal antibody has bound; a second monoclonal antibody to an epitope of the glycoprotein BCA-225 which antibody is linked to peroxidase; a wash solution for removing uncomplexed glycoprotein and uncomplexed second monoclonal antibody from the supports; an o-phenylenediamine substrate in appropriately buffered solution; hydrogen peroxide; and control samples of defined volume containing known amounts of glycoprotein.

Preferably, the first monoclonal antibody, and the blocking solution are already bound to the support and are not needed as separate items in the kit. For convenience, items which are voluminous or generally available may be excluded from the kit, e.g., components of the wash solution.

Assays which simply determine the presence or absence of the glycoprotein BCA-225 in a body fluid sample from a subject may also be useful for diagnosing or monitoring human breast cancer, if, as presently contemplated, the glycoprotein is only present in samples from subjects who have breast cancer.

The presence or absence of the glycoprotein may be determined directly or indirectly. Examples of direct methods include electrophoresis, e.g., two-dimensional electrophoresis. Indirect methods include immunoassays in which a body fluid sample from a subject is contacted with an antibody which specifically binds to the glycoprotein BCA-225 under conditions permitting formation of complex between the antibody and the glycoprotein; detecting the presence or absence of complex, e.g., by use of fluorescence polarization; and determining therefrom the presence or absence of the glycoprotein in the sample.

The following section "EXPERIMENTAL DETAILS" is set forth to aid in understanding of the present invention but is not intended, and should not be construed, to limit the invention as defined by the claims which follow thereafter.

EXPERIMENTAL DETAILS

Growth of T47D Clone 11 Cells and Collection of the Medium

The cells were grown in RPMI 1640 medium containing fetal calf serum (FCS) (10%), insulin (0.2 unit/ml), glutamine (2 mM), streptomycin (100 ug/ml), penicillin (100 units/ml), and mycostatin (25 ug/ml). For virus collection, the cells were seeded in RPMI 1640 medium containing 10% fetal calf serum for 24 hrs. followed by medium containing 5% dialyzed FCS and 1 nM 17-estradiol. After one medium change, this medium was replaced by one containing 10 nM progesterone free of (FCS) (day 5 after passage). From day 6, medium was collected every day, and the cells were refed with progesterone-containing medium. The collected medium was clarified by centrifugation for 15 min. (480 x g at 4°C) and stored at -70°C.

Isolation of Virus-like Particles from T47D Clone 11 Culture Medium

Approximately 8-12 liters of T47D clone 11 culture medium is concentrated to 200 ml using the Pellicon cassette system (Millipore) with a 100,000 molecule weight cut-off filter. After centrifugation at 100,000 x g for 90 min. the supernatant, designated Sup-100, is separated from the pellet that is then dissolved in TNE (0.01 M Tris HCl, pH 8.3/0.15 M NaCl/2 mM EDTA) and layered over a linear 20% sucrose gradient and centrifuged at 100,000 x g for 16 hr. at 4°C. The fractions are collected in three regions according to density (I.: 1.090-1.159 g/ml, II.: 1.160-1.190 g/ml, III.: 1.191-1.239 g/ml which is the Viral Region (VR) fragment) that are diluted and centrifuged as above for 90 min. to pellet the virus-like particles present. The pellets are resuspended in PBS and analyzed by Western blot and EIA for specific antigen content. The amount of total protein recovered in the region with a density corresponding to virus-like particles is about 1.5 mg per liter of culture medium.

Purification of Breast Tumor Antigen from the 100,000 x g Supernatant (Sup-100) of T47D Clone 11 Culture Medium

Sup-100 is fractionated with solid ammonium sulfate and the fraction precipitating between 50 and 80% ammonium sulfate saturation (Sup-100 AS) is collected by centrifugation (10,000 rpm, 30 min in Sorvall RC2B) and dialyzed against TNA buffer (0.05 M Tris HCl, pH 7.4/ 0.05 M NaCl/ 0.02% $NaN_3$). The precipitate formed during dialysis is removed by centrifugation and the supernatant is loaded on a Sephacryl-S-300 column equilibrated with TNA buffer. The column is eluted with the same buffer and the fractions are tested for BCA-225 using a monoclonal "sandwich" EIA. The fractions with high antigen content, corresponding to the first protein peak following void volume, are pooled and dialyzed against 0.05 M sodium phosphate, pH 7.0, containing 0.2 M NaCl and 0.02% $NaN_3$. The dialysate is then applied to a wheat germ lectin-Sepharose 6MB affinity column equilibrated with the dialyzing buffer. After washing the column with 15 volumes of buffer the antigen is eluted with N-acetyl-d-glucosamine (100 mg/ml buffer) and the fractions containing antigen are pooled, concentrated and dialyzed with PBS. This procedure results in about 70-fold enrichment of BCA-225 over Sup-100 AS. Analysis by SDS-PAGE Western transfer and immunoblotting reveals the presence of two molecular weight species (225 and 250 kd) both recognized by each of the three monoclonal antibodies (CU18, CU46 and CU26), and hereinafter designated BCA-225.

## Affinity Chromatography with CU18

CU18 antibodies were coupled to CNBr-activated Sepharose 4B (Pharmacia) (10 mg IgG/ml gel) following manufacturer's instructions. The unbound active sites on the gel were blocked with 1 M ethanolamine (pH 9.0). The partially purified antigen preparation from Sephacryl-S-300 chromatography was passed through the gel that was subsequently washed with low pH (4.0) and high pH (8.0 buffers). The bound antigen was eluted with 4 M NaCNS and was dialyzed against PBS.

## Further Characterization of BCA-225

The molecular weight of the antigen recognized by monoclonal antibody CU18 was determined by sodium duodecyl sulfate-polyacrylamide gel electrophoresis (SDS-PAGE) of the Sephacryl-S-300 purified fraction, followed by electrophoretic transfer to nitrocellulose membranes (Western blot), followed by CU18, biotinylated horse anti-mouse IgG avidin-biotin peroxidase complex (ABC) and $H_2O_2$-diaminobenzidine. The resulting two bands at the top of the gel were calculated to be approximately 250 and 225 Kd respectively by comparison with molecular weight standards run on an adjacent lane. Similar bands are observed with CU46 and CU26. A composite Western blot illustrating the identification of these two bands by the three monoclonal antibodies (CU18, CU26 and CU46) and the polyclonal antibodies (R402 and R406) used in the five-layer EIA has been prepared. This blot had two lanes, one containing the partially purified antigen (from Sephacryl-300 chromatography) and one containing SUP-100 AS. Whereas the monoclonal antibodies recognized only the 225, 250 Kd bands, the polyclonal rabbit antibodies recognized other molecular weight species in the crude antigen preparation SUP-100 AS.

## Glycoprotein Nature of BCA-225

As previously mentioned, BCA-225 binds to a wheat germ agglutinin affinity column suggesting an abundance of N-acetylglucosamine or N-acetylgalactosamine moieties in the carbohydrate portion of the protein. Further confirmation consists of the identification of the two protein bands by Western blot using a wheat germ lectin-peroxidase conjugate. Although the isoelectric point of BCA-225 has not yet been determined, its very strong binding to a chromatofocusing gel suggests that BCA-225 is acidic in nature.

## Amino Acid Composition of CU18 Affinity Purified Antigen

Amino acid analysis distribution shown below is a weight average of the two bands of affinity purified BCA-225:

|     |      |
| --- | ---- |
| ASP | 9.2% |
| THR | 6.4  |
| SER | 6.0  |
| GLU | 12.1 |
| PRO | 5.6  |
| GLY | 5.0  |
| ALA | 7.5  |
| CYS | 3.8  |

| | |
|---|---|
| VAL | 7.3 |
| MET | 0.8 |
| ILE | 3.1 |
| LEU | 10.1 |
| TYR | 3.3 |
| PHE | 4.3 |
| HIS | 2.8 |
| LYS | 8.4 |
| ARG | 4.0 |

Generation of Hybridomas and Production of Monoclonal Antibodies

1.        Immunization

Six 7-week old Balb/c mice were injected intraperioneally (i.p.) with 15-30 ug of antigen (VR or Sup-100 AS) in 300 ul of 50% Freund complete adjuvant-PBS. Seven days later each mouse was boosted (i.p.) with the same dose of antigen in 50% Freund incomplete adjuvant. The final boost containing 10 ug of antigen in 300 ul of PBS is given i.v. 7-24 days later. Three days after the i.v. injection the spleens are removed aseptically by blunt dissection for fusion.

2.        Fusion

The spleens are minced by pressing through a collector tissue sieve into a Petri dish containing RPMI 1640. After separation of connective tissue debris by sedimentation, the cell suspension in the supernatant is centrifuged at 2,000 rpm for 5 min. and the cells are washed twice in RPMI 1640. NS-1 murine myeloma cells are combined with the splenocytes (5:1, spleen:NS-1), centrifuged for 5 min. at 2,000 rpm and, after removal of the supernatant, 1 ml of 50% PEG solution at 37°C is slowly added over 1 min. with continuous shaking. The test tube is then gently swirled (90 seconds) in a 37° water bath and RPMI 1640 is slowly added according to the following sequence: 1 ml. (30 seconds), 3 ml (30 seconds), 16 ml (1 min.) and 10 ml. (30 seconds). The suspension is then allowed to stand at room temperature (5 min.), and after centrifugation (10 min. at 1,000 rpm) the supernatant is immediately removed and the cells are concentrated at $2 \times 10^0$ cells/ml by gentle addition of culture medium containing 15% pretested FCS. One hundred ul of the cell suspension is introduced into each of the 60 internal wells of a 96-well microtiter plate. The plates are incubated at 37° in 7% $CO_2$ for 4-18 hours before 100 ul of HAT (hypoxanthine, thymidine, aminopterin) medium is added to each well. Every third day 100 ul of culture supernatant is removed and replaced with an equal volume of HAT medium until hybridomas cover approximately 30% of each well, when the HAT medium is replaced every other day. At 50% confluency, 150 ul of culture supernatant is removed for initial screening by immunocytochemistry (see below).

3.    Cloning

Hybridomas are transferred to 24-well plates and HAT medium is gradually replaced, over 4 days, with RPMI 1640-15% FCS. Twenty ul of a hybridoma cell suspension (1,000 cells/ml) is serially diluted in a 96-well culture plate containing cell-free spent NS-1 medium (200 ul/well). One-half of the volume of each well is replaced with RPMI 1640-15% FCS on the fourth day and then every other day as clones increase in size. At 30% confluency, 150 ul of supernatant is removed, from wells which contain the lowest dilution of hybridoma cells, to test for Ig production and the cells in the wells with a positive result are recloned.

## Immunocytochemical Screening of Hybridomas and Characterization of Monoclonal Antibodies

1.          Screening

Since initially pure antigen was not available, screening by EIA with impure antigen presented the very real possibility that the antigen of interest may be present in amounts too small to be detected by the antibodies in the hybridoma supernatants to be tested and thus the initial screening of hybridomas is done by immunocytochemistry on sections cut from a Bouin-fixed, paraffin-embedded cell block of T47D clone 11 cells. Supernatants are initially tested in groups of five with subsequent testing of the individual supernatants from those groups that give a positive result. A secondary screening is done with "composite" sections consisting of several relevant tissues embedded in one paraffin block, e.g., two breast carcinomas, a fibroadenoma, normal breast, cystic disease and a T47D clone 11 cell block. These composite sections provide a great deal of information and permit a rapid initial evaluation of hybridomas leading to decisions that ultimately conserve considerable time and effort.

2.          Immunocytochemical Methods

The methods used are the indirect immunoperoxidase procedure and the avidin-biotin complex (ABC) method as recently described (Mesa-Tejada, R. In: MONOCLONAL ANTIBODIES AND BREAST CANCER, R.L. Ceriani, ed., Martinus-Nijhoff, Boston (1985)). The preparation of the reagents, including the peroxidase-conjugated antibodies used in both ICC and EIA (see below), are prepared according to previously described procedures (Mesa-Tejada, R. et al., J. Histochem. Cytochem. 26: 532 (1978)).

Table 1 summarizes the immunocytochemical results obtained with monoclonal antibodies CU18 on 289 different tissues, including 178 breast carcinomas, 60 nonbreast malignancies, 33 normal and benign breast tissues and 18 other normal tissues. A positive reaction was observed in 94% of the breast carcinomas in which the staining pattern was mainly intracytoplasmic although reaction product could also be observed on the cell membrane and lining small intracellular vacuolar structures. In carcinomas sufficiently differentiated to form glandular structures reaction product was also noted along the glycocalyx or apical surface of the cell. This glycocalyx type of staining reaction was also seen in 62% of 34 benign breast tissues as a fine, frequently weak, linear stain on the luminal surface of some, but not all ducts and in isolated acini of occasional lobules, both morphologically normal and atypical. In the series of 60 nonbreast carcinomas, an apical or glycocalyx reaction was noted in five of thirteen kidney carcinomas and in all six adenocarcinomas of the lung tested. A focal weak intracellular localization was also noted in one of eight squamous cell carcinomas of the lung.

The reaction with kidney carcinomas appears to be due to a chance crossreactivity with Tamm-Horsfall protein, a renal epithelial glycoprotein, suggested by the pattern of staining also observed in normal kidney tubules, and other immunochemical evidence. The reaction in the adenocarcinomas of the lung may be related to the identical surface stain seen focally in normal lung aveoli, in the granular or type II pneumocytes. It has not yet been determined whether the antigen localized on the apical surfaces of these histogenetically and functionally different cells is BCA-225 or a different antigen(s) structurally related through a similar or identical epitope recognized by CU18. Nevertheless, the strong cytoplasmic stain produced by CU18 in breast carcinomas suggest that its corresponding antigen is more likely to be found in the circulation of patients with invasive breast cancer, and that its detection in serum with CU18, CU46 and CU26 will preferentially reflect the presence of this disease.

## TABLE 1

IMMUNOPEROXIDASE STAIN OF HUMAN TISSUES

| | CU-18 |
|---|---|
| Breast Carcinomas | 167/178 (94%)* |
| Other Carcinomas | |
| Colon (7) | --- |
| Lung | |
| Squamous (8) | 1/8 |
| Adenocarcinoma (6) | 6/6** |
| Kidney (13) | 5/13*** |
| Stomach (3) | --- |
| Ovary (2) | --- |
| Prostate (6) | --- |
| U. Bladder (3) | --- |
| Hepatoma (2) | --- |
| Pancreas (2) | --- |
| Thyroid (5) | --- |
| Parotid (3) | --- |
| Normal and Benign Breast | |
| Tissues | 21/33 (62%)** |
| Fibroadenoma (6) | --- |
| Cystic disease (17) | 14/17 |
| Normal (5) | 3/5 |
| Lactating (3) | --- |
| Juvenile Hypertrophy (5) | 1/5 |
| Normal Kidney | 13/13 (100%)*** |
| Normal Lung | 5/5 (100%)** |

---

| | |
|---|---|
| * | Number positive/total (%) |
| ** | Apical or glycocalyx distribution |
| *** | Same distribution as Tamm-Horsfall protein |
| **** | Numbers in parentheses indicate the number of tissues tested |

Enzyme Immunoassay for the Detection of BCA-225

Three enzyme-linked immunosorbent assays (ELISA) have been developed to detect or quantitate BCA-225 using monoclonal and polyclonal antibodies to VR or Sup-100 antigens.

1.        Direct ELISA

Immulon 2 microtiter plates (Dynatech Laboratories) are coated with antigen in carbonate-bicarbonate buffer (0.05 M, pH 9.5) and incubated overnight at room temperature. The unreacted sites in the wells are blocked with 5% normal goat serum in the same buffer for one hour at 37°C. After washing with PBS containing 0.05% Tween 20, the wells are incubated with monoclonal antibody (CU18, CU46 or CU26) in PBS for 2 hr. at 37°C. After washing the plate with PBS-Tween, biotinylated horse anti-mouse IgG is added (2 hr. at 37°C) followed, after washing, with peroxidase labelled biotin-avidin complex (Vector Laboratories) for 1 hr. at 37°C. The plates are developed with o-phenylenediamine-$H_2O_2$ substrate at pH 6.0 and are read at 492 nm in an automated spectrophotometer.

2.        5-Layer Monoclonal-Polyclonal ELISA

The plates are coated with monoclonal antibody (e.g. 5 ug/well in 0.05 M carbonate-bicarbonate buffer, pH 9.5) with overnight incubation and the remaining reactive sites are blocked as described above. After washing with PBS-Tween the sample containing BCA-225 is added (2 hr. at 37°C) followed by a wash and incubation with rabbit anti-VR (R402 or R406) for 2 hr. at 37°C. Biotinylated goat anti-rabbit IgG (1 hr. at 37°C) is then followed by ABC and developed as described above.

Monoclonal "Sandwich" ELISA

The plates are coated with CU18 (purified from ascites, 25 ug/ml coating buffer, 200 ul/well and are blocked with 2% bovine serum albumin (BSA). After washing the sample containing BCA-225 in diluent (1% BSA, 1% mouse serum, in PBS with 0.05% Tween-20) is added and incubated overnight at room temperature. After washing, peroxidase conjugated CU46 (10 ug/ml, 200 ul/well) is added and incubated for 2 hr. at 37°C. After washing the plate is developed and read as described above.

Detection of BCA-225 in Human Serum

The monoclonal "sandwich" ELISA described above has been found to be the most reliable and reproducible method for detecting BCA-225 in human serum. Standards are prepared using pooled serum from healthy controls diluted at 1:2 and 1:10 with diluent buffer. Standards containing less than 1 ng/200 ul up to 200 ng/200 ul of the Sephacryl-S-300 antigen with BCA-225 activity, are used to construct a reference curve to quantitate the readings obtained in patients' samples. The patients' serum or plasma samples are diluted 1:2 and 1:10 and added to the plate in duplicate. Figure 2 illustrates a typical reference standard curve obtained with this assay using serum samples.

Clinical Study Correlating the Concentration of BCA-225 in Serum with the Presence and Status of Human Breast Cancer

Figure 3 illustrates the concentration of BCA-225 antigen obtained in the serum of a series of 433 total patients subdivided into seven clinically relevant groups as indicated. The circled numbers at the bottom of each column are the "0" values in that group, i.e., those patients in whose sera BCA-225 could not be detected.

The dotted line at 250 ng/ml in Figure 3 represents an arbitrary boundary between the "positive" (above) and "negative" (below) values. This value was arbitrarily chosen because it is approximately equal to the mean plus two standard deviations of the "Benign Breast Disease" group (second column). See Table 2.

## TABLE 2

| Group | Number | Mean ± S.D. |
|---|---|---|
| Breast Cancer Patients | | |
| Stage 0-I | 32 | 346 ± 91.2 |
| II | 52 | 997 ± 296.6 |
| III-IV | 41 | 1825 ± 419.3 |
| No clinical evidence of active disease | 114 | 54 ± 1.4 |
| Benign Breast Disease | 69 | 189 ± 21.5 |
| Other Cancers | 23 | 58 ± 27.2 |
| Normal Healthy Controls | 101 | 14 ± 5.3 |

However, the normal values should more correctly be calculated from the range of each of the control groups (the mean plus two standard deviations) as follows:

## TABLE 3

Normal Values of BCA-225

| Group | Normal Range |
|---|---|
| Normal Healthy Controls | 0 - 25 |
| Benign Breast Disease | 0 - 233 |
| Other Cancer | 0 - 113 |

Thus, the proportion of breast cancer patients with abnormal BCA-225 values can be compared with each group of controls, as follows:

## TABLE 4

| STAGE | Patients with Abnormal BCA-225 Values Compared to: | | |
|---|---|---|---|
| | Normals | Benign Disease | Other Cancer |
| 0-I | 26/32 (81%) | 17/32 (53%) | 21/32 (66%) |
| II | 40/52 (77%) | 27/52 (52%) | 37/52 (71%) |
| III | 9/10 (90%) | 7/10 (70%) | 8/10 (80%) |
| IV | 28/31 (90%) | 26/31 (83%) | 28/31 (90%) |
| TOTAL | 103/125(82%) | 77/125(62%) | 94/125(75%) |

## Daunorubicin-Conjugated Monoclonal Antibody to Human Breast Carcinoma:  In Vitro Interraction with T47D Breast Carcinoma Cells

Daunorubicin (DNR), an anthracycline chemotherapeutic agent active in breast cancer, was conjugated to monoclonal antibody CU18 by periodate oxidation and Schiff- base formation.  CU18, which recognizes an antigen found on the surface and cytoplasm of T47D human breast carcinoma cells, retained its ability to bind antigen after conjugation, demonstrated by whole-cell radioim- mune binding assay and by indirect immunoperoxidase staining of tissue sections.  Taking advantage of DNR autofluorescence, the uptake and intracellular distribution of CU18-DNR by clone 11 T47D cells was observed by digitized video fluorescent microscopy and compared, at equal concentrations, with free DNR and an irrelevant conjugate 225-DNR (anti-melanoma).  CU18-DNR was rapidly taken up by the cells in suspension and distributed on the membrane and in the cytoplasm at $4^o$ and $37^o$.  At $4^o$ uptake of free DNR was imperceptible in live cells while at $37^o$ faint cytoplasmic fluorescence was noted.  Intracellular uptake of 225-DNR was also noted at both temperatures, but not the prominent "Golgi-like" localization of CU18-DNR.  Finally, addition of fluoresceinated anti-mouse IgG demonstrated strong granular membrane fluorescence only in the T47D cells previously exposed to CU18-DNR.  These results substantiate previous reports that specificity is not an absolute requirement for the uptake of antibody by malignant cells and indicates the need for caution in the planning and interpretation of such experiments as well as for further study of antibody-uptake mechanisms.  Nevertheless, the demonstrated enhanced uptake of CU18-DNR as compared to equimolar amounts of free

0221561

DNR indicates considerable potential for the use of these conjugates as specifically directed immunotherapeutic agents. Preliminary cytoxicity data supports this conclusion.

Immunocytochemical Identification of Cutaneous Breast Cancer Metastases with Monoclonal Antibody CU18

Monoclonal antibody CU18, prepared from shed products of the T47D human breast carcinoma cell line, is capable of immunocytochemically discriminating cells of breast cancer origin with considerable sensitivity and specificity. The intracellular localization of antigen defined by this antibody has been found to reliably identify malignant cells of mammary epithelial origin. The finding of strong reactivity in a metastatic lesion in the skin of a patient with disseminated breast carcinoma prompted us to compile a series of 19 such cases in which paraffin sections of the original breast carcinoma were also available. Twelve cutaneous metastases from different carcinomas (6 colon, 2 rectum, 2 stomach, 2 lung) were also stained with CU18 using an indirect immunoperoxidase method. A positive reaction was noted in all 19 primary breast carcinomas and in all but one corresponding metastases. In two cases a weak, focal reaction was seen in both the primary and the metastatic lesion. In two other cases such a weak reaction was noted only in either the primary or the metastasis. In the control group, ten of the twelve cases were negative and two gave a very weak, focal positive reaction. These results indicate that even though the intracellular localization of the CU18-defined antigen is highly specfific for breast carcinoma and its corresponding metastatic lesions, very weak and focal intracellular reactions can also be observed in malignant cells of different histogenesis and such reactions, therefore, should be interpreted with considerable caution.

0221561

WHAT IS CLAIMED IS:

1.      An isolated glycoprotein useful as an indicator of human breast cancer or metastases thereof, which glycoprotein is derived from human breast carcinomas, is a molecular species of about 225-250 kilodaltons, and specifically binds to the monoclonal antibody produced by the hybridoma cell line having ATCC Accession No. HB 8936 (CU26).

2.      The glycoprotein of claim 1 to which a marker is bound.

3.      The glycoprotein of claim 2, wherein the marker is an enzyme which catalyzes a reaction which yields a detectable product.

4.      The glycoprotein of claim 3, wherein the enzyme is horseradish peroxidase.

5.      The glycoprotein of claim 2, wherein the marker is a radioactive label.

6.      The glycoprotein of claim 5, wherein the label is $^{125}I$.

7.      The glycoprotein of claim 2, wherein the marker is a fluorescent label.

8.      The glycoprotein of claim 7, wherein the label is fluorescein isothiocyanate.

9.      The glycoprotein of claim 7, wherein the label is rhodamine.

0221561

10.    The glycoprotein of claim 2, wherein the marker is a magnetic entity.

11.    The glycoprotein of claim 10, wherein the magnetic entity is copper.

12.    The glycoprotein of claim 10, wherein the magnetic entity is iron.

13.    The glycoprotein of claim 2, wherein the marker is an imageable entity.

14.    The glycoprotein of claim 13, wherein the marker is a radionuclide.

15.    The glycoprotein of claim 14, wherein the radionuclide is $^{111}$In.

16.    A hybridoma which produces a monoclonal antibody having binding specificity to the glycoprotein of claim 1, wherein the hybridoma is the cell line having ATCC Accession No. HB 8936 (CU26).

17.    A monoclonal antibody which specifically binds to the glycoprotein of claim 1 and is produced by the hybridoma cell line having ATCC Accession No. HB 8936 (CU26).

18.    A monoclonal antibody of claim 17, which specifically binds to the glycoprotein of claim 1 and to which a marker is bound.

19.    A monoclonal antibody which specifically binds to the glycoprotein of claim 1 to which a magnetic entity is bound.

20.    A monoclonal antibody of claim 19, wherein the magnetic entity is copper.

21.    A monoclonal antibody of claim 19, wherein the magnetic entity is iron.

22.    An assay for diagnosing human breast cancer in a subject which comprises quantitatively determining in a body fluid sample of defined volume from the subject the amount of the glycoprotein of claim 1 present in the sample, comparing the amount so determined with predetermined amounts indicative of the presence or absence of human breast cancer in samples of the same volume and thereby diagnosing the presence or absence of human breast cancer in the subject.

23.    An assay in accordance with claim 22, wherein the body fluid sample is a serum sample.

24.    An immunoassay for diagnosing human breast cancer in a subject which comprises quantitatively determining in a body fluid sample of defined volume from the subject the amount of the glycoprotein of claim 1 present in the sample by contacting the sample with an antibody which specifically binds to the glycoprotein and comparing the amount so determined with predetermined amounts indicative of the presence or absence of human breast cancer in samples of the same volume, and thereby diagnosing the presence or absence of human breast cancer in the subject.

25.    An immunoassay for diagnosing human breast cancer in accordance with claim 24, wherein the antibody is bound to a marker.

26. An immunoassay for diagnosing human breast cancer which comprises:

    (a) immobilizing on a support, a monoclonal antibody which specifically binds to an epitope on the glycoprotein of claim 1;

    (b) contacting the immobilized monoclonal antibody with a body fluid sample of defined volume from a subject under conditions permitting formation of a complex between the monoclonal antibody and the glycoprotein;

    (c) removing uncomplexed sample components; and

    (d) quantitatively determining the amount of the glycoprotein present;

    (e) comparing the amount so determined with predetermined amounts indicative of the presence or absence of human breast cancer in samples of the same volume; and

    (f) diagnosing the presence or absence of human breast cancer in the subject.

27. An immunoassay in accordance with claim 26, wherein in step (d) the amount of the glycoprotein present in the sample is determined by:

    (i) contacting the complex of monoclonal antibody and glycoprotein with a detectable monoclonal antibody which specifically binds to a different epitope on the glycoprotein under conditions permitting the binding of the detectable monoclonal antibody to the complex;

(ii) removing the unbound detectable monoclonal antibody; and

(iii) quantitatively determining the amount of bound detectable monoclonal antibody, and thereby the amount of glycoprotein in the complex.

28. An immunoassay in accordance with claim 27, wherein the detectable antibody is an antibody linked to an enzyme which catalyzes a reaction which yields a detectable product.

29. An immunoassay in accordance with claim 28, wherein the enzyme is horseradish peroxidase.

30. An immunoassay in accordance with claim 27, wherein the detectable antibody is a radioactively labelled antibody.

31. An immunoassay in accordance with claim 30, wherein the antibody is labelled with $^{125}I$.

32. An immunoassay in accordance with claim 27, wherein the detectable antibody is a fluorescently labelled antibody.

33. An immunoassay in accordance with claim 32, wherein the antibody is labelled with fluorescein isothiocyanate.

34. An immunoassay in accordance with claim 32, wherein the antibody is labelled with rhodamine.

35. An immunoassay in accordance with claim 27, wherein the detectable antibody is a magnetically labelled antibody.

36. an immunoassay in accordance with claim 35, wherein the antibody is labelled with copper.

37. An immunoassay in accordance with claim 35, wherein the antibody is labelled with iron.

38. An immunoassay in accordance with claim 27, wherein the detectable antibody is linked to an imageable entity.

39. A method for diagnosing human breast cancer in a subject which comprises contacting a body fluid sample of defined volume from the subject with an antibody to the glycoprotein of claim 1 under suitable conditions such that a detectable complex forms if the glycoprotein is present in an amount indicative of human breast cancer and detecting the complex.

40. A method for monitoring human breast cancer in a subject which comprises quantitatively determining at various times in body fluid samples of defined volume from the subject the amount of the glycoprotein of claim 1 present in the samples and comparing the amounts so determined.

41. A method for monitoring human breast cancer in a subject in accordance with claim 40, wherein the amount of glycoprotein present in the sample is determined by contacting the sample with a detectable antibody which specifically binds to the glycoprotein.

42. A monoclonal antibody which specifically binds to the glycoprotein of claim 1, and which is linked to a chemotherapeutic agent or toxin.

43. A monoclonal antibody of claim 42, wherein the chemotherapeutic agent or toxin is carried in a liposome bound to the antibody.

44. A method of delivering a chemotherapeutic agent or toxin to a cancer cell which comprises contacting the cell with a monoclonal antibody of claim 42.

45. A method of claim 44, wherein the cancer cell is a human breast cancer cell.

46. A monoclonal antibody which specifically binds to the glycoprotein of claim 1 to which an imageable entity is bound.

47. A monoclonal antibody of claim 46, wherein the imageable entity is a radionuclide.

48. A monoclonal antibody of claim 47, wherein the radionuclide is $^{111}$In.

49. A method of imaging a cancer cell which comprises contacting the cell with a monoclonal antibody of claim 46 and detecting the imageable entity.

50. An immunoassay for diagnosing human breast cancer in a subject which comprises:

    (a) immobilizing on a support a monoclonal antibody having binding sites which specifically bind to an epitope on the glycoprotein of claim 1;

(b)   contacting the immobilized monoclonal antibody with a body fluid sample of defined volume from a subject and a known amount of labelled glycoprotein, which amount is sufficient to bind to substantially all of the binding sites of the immobilized monoclonal antibody under conditions permitting the formation of a complex between the monoclonal antibody, labelled glycoprotein and unlabelled glycoprotein in the sample;

(c)   removing components of the sample and labelled glycoprotein not present in the complex;

(d)   quantitatively determining the amount of labelled glycoprotein in the complex;

(e)   determining therefrom the amount of unlabelled glycoprotein present in the complex and thereby the amount in the sample;

(f)   comparing the amount of unlabelled glycoprotein so determined with predetermined amounts indicative of the presence or absence of human breast cancer in samples of the same defined volume; and

(g)   thus diagnosing the presence or absence of human breast cancer in the subject.

51.   A kit for performing the immunoassay of claim 27 which comprises:

(a)   a support;

(b) a first monoclonal antibody directed to one epitope of the glycoprotein;

(c) a blocking solution to block sites on the support where no first monoclonal antibody has bound;

(d) a second monoclonal antibody to a different epitope of the glycoprotein to which peroxidase is bound;

(e) a wash solution for removing uncomplexed glycoprotein and uncomplexed second monoclonal antibody from the support;

(f) o-phenylenediamine in an appropriately buffered solution;

(g) hydrogen peroxide; and

(h) control samples of defined volume containing known amounts of the glycoprotein.

52. A kit according to claim 51, wherein the first monoclonal antibody and blocking solution are bound to the support.

53. An assay for diagnosing human breast cancer in a subject which comprises determining the presence or absence of the glycoprotein of claim 1 in a body fluid sample from the subject.

54. An assay of claim 53, wherein the body fluid serum.

55. An assay of claim 53, wherein the presence or absence of glycoprotein is determined by two dimensional electrophoresis.

56. An immunoassay for diagnosing human breast cancer in a subject which comprises contacting a body fluid sample from the subject with an antibody which specifically binds to the glycoprotein of claim 1 under conditions permitting formation of a complex between the antibody and the glycoprotein, detecting the presence or absence of the complex, determining therefrom the presence or absence of the glycoprotein and thereby diagnosing human breast cancer.

57. An immunoassay of claim 56, wherein the presence or absence of the complex is determined by fluorescence polarization.

## FIGURE 1

FIGURE 2

FIGURE 3

FIGURE 4

Fraction no.

FIGURE 5

FIGURE 6

CONCENTRATION UNLABELLED AG, NG/ML